# EUROPEAN PATENT SPECIFICATION

(11) **EP 1 729 807 B1**
(45) Date of publication and mention of the grant of the patent: **11.11.2009**
(21) Application number: 05729377.1
(22) Date of filing: 22.03.2005
(51) Int. Cl.: A61K 45/06, A61K 31/337, A61K 31/517, A61P 35/00

(54) **COMBINATION THERAPY WITH AZD-2171**
KOMBINATIONSTHERAPIE MIT AZD-2171
THERAPIE DE COMBINAISON AVEC AZD-2171

(30) Priority: 23.03.2004 GB 0406445
(43) Date of publication of application: 13.12.2006
(73) Proprietor: AstraZeneca AB, 151 85 Södertälje (SE)
(72) Inventor: Wedge, Stephen Robert, AstraZeneca R & D Alderley, Macclesfield Cheshire SK10 4TG (GB)
(86) International application number: PCT/GB2005/001089
(87) International publication number: WO 2005/092385

(56) References cited:
- WO-A-00/47212
- WO-A-2004/096224
- WO-A-2005/004871
- MALIK A K HANS-PETER GERBER: "Targeting VEGF ligands and receptors in cancer" TARGETS, ELSEVIER, vol. 2, no. 2, 1 April 2003 (2003-04-01), pages 48-57, XP004886645 ISSN: 1477-3627
- MARME D: "THE IMPACT OF ANTI-ANGIOGENIC AGENTS ON CANCER THERAPY" JOURNAL OF CANCER RESEARCH AND CLINICAL ONCOLOGY, SPRINGER INTERNATIONAL, BERLIN, DE, vol. 129, no. 11, 2003, pages 607-620, XP001182072 ISSN: 0171-5216

## Description

The present invention relates to the production of an antiangiogenic and/or vascular permeability reducing effect in a warm-blooded animal such as a human which is optionally being treated with ionising radiation, particularly to the treatment of a cancer, particularly a cancer involving a solid tumour, by means of the administration of AZD2171 in combination with a taxane; to a pharmaceutical composition comprising AZD2171 and a taxane; to a combination product comprising AZD2171 and a taxane for use in a method of treatment of a human or animal body by therapy; to a kit comprising AZD2171 and a taxane; to the use of AZD2171 and a taxane in the manufacture of a medicament for use in the production of an antiangiogenic and/or vascular permeability reducing effect in a warm-blooded animal such as a human which is optionally being treated with ionising radiation.

Formal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Alteration of vascular permeability is thought to play a role in both normal and pathological physiological processes (Cullinan-Bove et al, 1993, Endocrinology 133: 829-837; Senger et al, 1993, Cancer and Metastasis Reviews, 12: 303-324). Several polypeptides with in vitro endothelial cell growth promoting activity have been identified including, acidic and basic fibroblast growth factors (aFGF & bFGF) and vascular endothelial growth factor (VEGF). By virtue of the restricted expression of its receptors, the growth factor activity of VEGF, in contrast to that of the FGFs, is relatively specific towards endothelial cells. Recent evidence indicates that VEGF is an important stimulator of both normal and pathological angiogenesis (Jakeman et al, 1993, Endocrinology, 133: 848-859; Kolch et al. 1995, Breast Cancer Research and Treatment, 36:139-155) and vascular permeability (Connolly et al, 1989, J. Biol. Chem. 264: 20017-20024). Antagonism of VEGF action by sequestration of VEGF with antibody can result in inhibition of tumour growth (Kim et al, 1993, Nature 362: 841-844).

Receptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity which leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt-1 (also referred to as VEGFR-1), the kinase insert domain-containing receptor, KDR (also referred to as VEGFR-2 or Flk-1), and another fms-like tyrosine kinase receptor, Flt-4. Two of these related RTKs, Flt-1 and KDR, have been shown to bind VEGF with high affinity (De Vries et al, 1992, Science 255: 989-991; Terman et al, 1992, Biochem. Biophys. Res. Comm. 1992, 187: 1579-1586). Binding of VEGF to these receptors expressed in heterologous cells has been associated with changes in the tyrosine phosphorylation status of cellular proteins and calcium fluxes.

VEGF is a key stimulus for vasculogenesis and angiogenesis. This cytokine induces a vascular sprouting phenotype by inducing endothelial cell proliferation, protease expression and migration, and subsequent organisation of cells to form a capillary tube (Keck, P.J., Hauser, S.D., Krivi, G., Sanzo, K., Warren, T., Feder, J., and Connolly, D.T., Science (Washington DC), 246: 1309-1312, 1989; Lamoreaux, W.J., Fitzgerald, M.E., Reiner, A., Hasty, K.A., and Charles, S.T., Microvasc. Res., 55: 29-42, 1998; Pepper, M.S., Montesano, R., Mandroita, S.J., Orci, L. and Vassalli, J.D., Enzyme Protein, 49: 138-162, 1996.). In addition, VEGF induces significant vascular permeability (Dvorak, H.F., Detmar, M., Claffey, K.P., Nagy, J.A., van de Water, L., and Senger, D.R., (Int. Arch. Allergy Immunol., 107: 233-235, 1995; Bates, D.O., Heald, R.I., Curry, F.E. and Williams, B. J. Physiol. (Lond.), 533: 263-272, 2001), promoting formation of a hyper-permeable, immature vascular network which is characteristic of pathological angiogenesis.

It has been shown that activation of KDR alone is sufficient to promote all of the major phenotypic responses to VEGF, including endothelial cell proliferation, migration, and survival, and the induction of vascular permeability (Meyer, M., Clauss, M., Lepple-Wienhues, A., Waltenberger, J., Augustin, H.G., Ziche, M., Lanz, C., Büttner, M., Rziha, H-J., and Dehio, C., EMBO J., 18: 363-374, 1999; Zeng, H., Sanyal, S. and Mukhopadhyay, D., J. Biol. Chem., 276: 32714-32719, 2001; Gille, H., Kowalski, J., Li, B., LeCouter, J., Moffat, B, Zioncheck, T.F., Pelletier, N. and Ferrara, N., J. Biol. Chem., 276: 3222-3230, 2001).

Quinazoline derivatives which are inhibitors of VEGF receptor tyrosine kinase are described in International Patent Application Publication No. WO 00/47212. AZD2171 is described in WO 00/47212 and is Example 240 therein. AZD2171 is 4-(4-fluoro-2-methyl-1H-indol-5-yloxy)-6-methoxy-7-(3-(pyrrolidin-1-yl)propoxy)quinazoline:

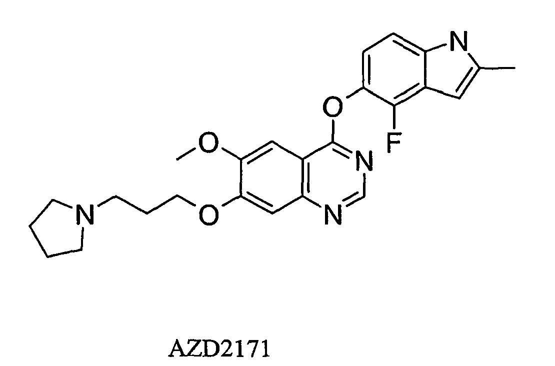

AZD2171 shows excellent activity in the in vitro (a) enzyme and (b) HUVEC assays that are described in WO 00/47212 (pages 80-83). The AZD2171 IC₅₀ values for inhibition of isolated KDR (VEGFR-2) and Flt-1 (VEGFR-1) tyrosine kinase activities in the enzyme assay were <2 nM and 5 ± 2 nM respectively. AZD2171 inhibits VEGF-stimulated endothelial cell proliferation potently (IC₅₀ value of 0.4 ± 0.2 nM in the HUVEC assay), but does not inhibit basal endothelial cell proliferation appreciably at a > 1250 fold greater concentration (IC₅₀ value is > 500 nM). The growth of a Calu-6 tumour xenograft in the in vivo solid tumour model described in WO 00/47212 (page 83) was inhibited by 49%^{**}, 69%^{***} and 91%^{***} following 28 days of once-daily oral treatment with 1.5, 3 and 6 mg/kg/day AZD2171 respectively (P^{**}<0.01, P^{***}<0.0001; one-tailed t test). AZD2171 has been shown to elicit broad-spectrum anti-tumour activity in a range of models following once-daily oral administration.

International Patent Application No. PCT/GB2004/005359 describes AZD2171 maleate salt and states that AZD2171 maleate salt may be applied as a sole therapy or may be used with one or more other substances and/or treatments. A list of other substances is given including taxoids of which Taxol® and Taxotere® are named.

In WO 00/47212 it is stated that compounds of the invention:
"may be applied as a sole therapy or may involve, in addition to a compound of the invention, one or more other substances and/or treatments. Such conjoint treatment may be achieved by way of the simultaneous, sequential or separate administration of the individual components of the treatment."
WO 00/47212 then goes on to describe examples of such conjoint treatment including surgery, radiotherapy and various types of chemotherapeutic agent,

Nowhere in WO 00/47212 is the specific combination of AZD2171 and a taxane suggested.

Nowhere in WO 00/47212 does it state that use of any compound of the invention therein with other treatments will produce surprisingly beneficial effects.

Unexpectedly and surprisingly we have now found that the particular compound AZD2171 used in combination with a particular selection from the combination therapies listed in WO 00/47212, namely with a taxane, produces significantly better effects than any one of AZD2171 and a taxane used alone. In particular, AZD2171 used in combination with a taxane produces significantly better effects on solid tumours than any one of AZD2171 and a taxane used alone.

Anti-cancer effects of a treatment of the present invention include, but are not limited to, anti-tumour effects, the response rate, the time to disease progression and the survival rate. Anti-tumour effects of a treatment of the present invention include but are not limited to, inhibition of tumour growth, tumour growth delay, regression of tumour, shrinkage of tumour, increased time to regrowth of tumour on cessation of treatment, slowing of disease progression. It is expected that when a treatment of the present invention is administered to a warm-blooded animal such as a human, in need of treatment for cancer involving a solid tumour, said treatment will produce an effect, as measured by, for example, one or more of: the extent of the anti-tumour effect, the response rate, the time to disease progression and the survival rate. Anti-cancer effects include prophylactic treatment as well as treatment of exiting disease.

A method for the production of an antiangiogenic and/or vascular permeability reducing effect in a warm-blooded animal such as a human may comprise administering to said animal an effective amount of AZD2171 or a pharmaceutically acceptable salt thereof, before, after or simultaneously with an effective amount of a taxane.

A method for the production of an antiangiogenic and/or vascular permeability reducing effect in a warm-blooded animal such as a human, may comprise administering to said animal an effective amount of AZD2171, or a pharmaceutically acceptable salt thereof excluding an AZD2171 maleate salt, before, after or simultaneously with an effective amount of a taxane.

A method for the treatment of a cancer in a warm-blooded animal such as a human, may comprise administering to said animal an effective amount of AZD2171 or a pharmaceutically acceptable salt thereof, before, after or simultaneously with an effective amount of a taxane.

A method for the treatment of a cancer in a warm-blooded animal such as a human, may comprise administering to said animal an effective amount of AZD2171, or a pharmaceutically acceptable salt thereof excluding an AZD2171 maleate salt, before, after or simultaneously with an effective amount of a taxane.

A method for the treatment of a cancer involving a solid tumour in a warm-blooded animal such as a human, may comprise administering to said animal an effective amount of AZD2171, or a pharmaceutically acceptable salt thereof, before, after or simultaneously with an effective amount of a taxane.

A method for the treatment of a cancer involving a solid tumour in a warm-blooded animal such as a human, may comprise administering to said animal an effective amount of AZD2171, or a pharmaceutically acceptable salt thereof excluding an AZD2171 maleate salt, before, after or simultaneously with an effective amount of a taxane.

A method for the production of an antiangiogenic and/or vascular permeability reducing effect in a warm-blooded animal such as a human, may comprise administering to said animal an effective amount of AZD2171 or a pharmaceutically acceptable salt thereof, before, after or simultaneously with an effective amount of a taxane; wherein AZD2171 and a taxane may each optionally be administered together with a pharmaceutically acceptable excipient or carrier.

A method for the production of an antiangiogenic and/or vascular permeability reducing effect in a warm-blooded animal such as a human, may comprise administering to said animal an effective amount of AZD2171, or a pharmaceutically acceptable salt thereof excluding an AZD2171 maleate salt, before, after or simultaneously with an effective amount of a taxane; wherein AZD2171 and a taxane may each optionally be administered together with a pharmaceutically acceptable excipient or carrier.

A method for the treatment of a cancer in a warm-blooded animal such as a human, may comprise administering to said animal an effective amount of AZD2171 or a pharmaceutically acceptable salt thereof, before, after or simultaneously with an effective amount of a taxane; wherein AZD2171 and a taxane may each optionally be administered together with a pharmaceutically acceptable excipient or carrier.

A method for the treatment of a cancer in a warm-blooded animal such as a human, may oomprise administering to said animal an effective amount of AZD2171, or a pharmaceutically acceptable salt thereof excluding an AZD2171 maleate salt, before, after or simultaneously with an effective amount of a taxane; wherein AZD2171 and a taxane may each optionally be administered together with a pharmaceutically acceptable excipient or carrier.

A method for the treatment of a cancer involving a solid tumour in a warm-blooded animal such as a human, may comprise administering to said animal an effective amount of AZD2171 or a pharmaceutically acceptable salt thereof, before, after or simultaneously with an effective amount of a taxane; wherein AZD2171 and a taxane may each optionally be administered together with a pharmaceutically acceptable excipient or carrier.

A method for the treatment of a cancer involving a solid tumour in a warm-blooded animal such as a human, may comprise administering to said animal an effective amount of AZD2171, or a pharmaceutically acceptable salt thereof excluding an AZD2171 maleate salt, before, after or simultaneously with an effective amount of a taxane; wherein AZD2171 and a taxane may each optionally be administered together with a pharmaceutically acceptable excipient or carrier.

According to the present invention there is provided a pharmaceutical composition which comprises AZD2171 or a pharmaceutically acceptable salt thereof, and a taxane in association with a pharmaceutically acceptable excipient or carrier.

According to a further aspect of the present invention there is provided a pharmaceutical composition which comprises AZD2171, or a pharmaceutically acceptable salt thereof excluding an AZD2171 maleate salt, and a taxane in association with a pharmaceutically acceptable excipient or carrier.

According to a further aspect of the present invention there is provided a combination product comprising AZD2171 or a pharmaceutically acceptable salt thereof and a taxane, for use in a method of treatment of a human or animal body by therapy.

According to a further aspect of the present invention there is provided a combination product comprising AZD2171 or a pharmaceutically acceptable salt thereof excluding an AZD2171 maleate salt, and a taxane, for use in a method of treatment of a human or animal body by therapy.

According to a further aspect of the present invention there is provided a kit comprising AZD2171 or a pharmaceutically acceptable salt thereof, and a taxane.

According to a further aspect of the present invention there is provided a kit comprising AZD2171 or a pharmaceutically acceptable salt thereof excluding an AZD2171 maleate salt, and a taxane.

According to a further aspect of the present invention there is provided a kit comprising:
a) AZD2171 or a pharmaceutically acceptable salt thereof in a first unit dosage form;
b) a taxane in a second unit dosage form; and
c) container means for containing said first and second dosage forms.

According to a further aspect of the present invention there is provided a kit comprising:
a) AZD2171 or a pharmaceutically acceptable salt thereof excluding an AZD2171 maleate salt, in a first unit dosage form;
b) a taxane in a second unit dosage form; and
c) container means for containing said first and second dosage forms.

According to a further aspect of the present invention there is provided a kit comprising:
a) AZD2171 or a pharmaceutically acceptable salt thereof, together with a pharmaceutically acceptable excipient or carrier, in a first unit dosage form;
b) a taxane together with a pharmaceutically acceptable excipient or carrier, in a second unit dosage form; and
c) container means for containing said first and second dosage forms.

According to a further aspect of the present invention there is provided a kit comprising:
a) AZD2171 or a pharmaceutically acceptable salt thereof excluding an AZD2171 maleate salt, together with a pharmaceutically acceptable excipient or carrier, in a first unit dosage form;
b) a taxane together with a pharmaceutically acceptable excipient or carrier, in a second unit dosage form; and
c) container means for containing said first and second dosage forms.

According to a further aspect of the present invention there is provided the use of AZD2171 or a pharmaceutically acceptable salt thereof and a taxane in the manufacture of a medicament for use in the production of an antiangiogenic and/or vascular permeability reducing effect in a warm-blooded animal such as a human.

According to a further aspect of the present invention there is provided the use of AZD2171 or a pharmaceutically acceptable salt thereof excluding an AZD2171 maleate salt, and a taxane in the manufacture of a medicament for use in the production of an antiangiogenic and/or vascular permeability reducing effect in a warm-blooded animal such as a human.

According to a further aspect of the present invention there is provided the use of AZD2171 or a pharmaceutically acceptable salt thereof and a taxane in the manufacture of a medicament for use in the production of an anti-casicer effect in a warm-blooded animal such as a human.

According to a further aspect of the present invention there is provided the use of AZD2171 or a pharmaceutically acceptable salt thereof excluding an AZD2171 maleate salt, and a taxane in the manufacture of a medicament for use in the production of an anti-cancer effect in a warm-blooded animal such as a human.

According to a further aspect of the present invention there is provided the use of AZD2171 or a pharmaceutically acceptable salt thereof and a taxane in the manufacture of a medicament for use in the production of an anti-tumour effect in a warm-blooded animal such as a human.

According to a further aspect of the present invention there is provided the use of AZD2171 or a pharmaceutically acceptable salt thereof excluding an AZD2171 maleate salt, and a taxane in the manufacture of a medicament for use in the production of an anti-tumour effect in a warm-blooded animal such as a human.

According to a further aspect of the present invention there is provided a combination treatment comprising the administration of an effective amount of AZD2171 or a pharmaceutically acceptable salt thereof, optionally together with a pharmaceutically acceptable excipient or carrier, and the simultaneous, sequential or separate administration of an effective amount of a taxane; wherein a taxane may optionally be administered together with a pharmaceutically acceptable excipient or carrier; to a warm-blooded animal such as a human in need of such therapeutic treatment.

According to a further aspect of the present invention there is provided a combination treatment comprising the administration of an effective amount of AZD2171 or a pharmaceutically acceptable salt thereof excluding an AZD2171 maleate salt, optionally together with a pharmaceutically acceptable excipient or carrier, and the simultaneous, sequential or separate administration of an effective amount of a taxane; wherein a taxane may optionally be administered together with a pharmaceutically acceptable excipient or carrier; to a warm-blooded animal such as a human in need of such therapeutic treatment. Such therapeutic treatment includes an antiangiogenic and/or vascular permeability effect, an anti-cancer effect and an anti-tumour effect.

A combination treatment of the present invention as defined herein may be achieved by way of the simultaneous, sequential or separate administration of the individual components of said treatment. A combination treatment as defined herein may be applied as a sole therapy or may involve surgery or radiotherapy or an additional chemotherapeutic agent in addition to a combination treatment of the invention. Surgery may comprise the step of partial or complete tumour resection, prior to, during or after the administration of the combination treatment with AZD2171 described herein.

Other chemotherapeutic agents for optional use with a combination treatment of the present invention include those described in WO 00/47212 which is incorporated herein by reference. Such chemotherapy may cover five main categories of therapeutic agent:
(i) other antiangiogenic agents including vascular targeting agents;
(ii) cytostatic agents;
(iii) biological response modifiers (for example interferon);
(iv) antibodies (for example edrecolomab); and
(v) antiproliferative/antineoplastic drugs and combinations thereof, as used in medical oncology; and other categories of agent are:
(vi) antisense therapies;
(vii) gene therapy approaches; and
(ix) immunotherapy approaches.

Particular examples of chemotherapeutic agents for use with a combination treatment of the present invention are raltitrexed, etoposide, vinorelbine, cisplatin, oxaliplatin, gemcitabine, irinotecan (CPT-11) and 5-fluorouracil (5-FU, (including capecitabine)) ; such combinations are expected to be particularly useful for the treatment of cancer of the lung, head and neck, colon, rectum, brain, thyroid, oesophagus, stomach, cervix, ovary, skin, breast, bladder and pancreas.

The administration of a triple combination of AZD2171, a taxane and ionising radiation may produce effects, such as anti-tumour effects, greater than those achieved with any of AZD2171, a taxane and ionising radiation used alone, greater than those achieved with the combination of AZD2171 and a taxtane, greater than those achieved with the combination of AZD2171 and ionising radiation, greater than those achieved with the combination of a taxane and ionising radiation.

A method for the production of an antiangiogenic and/or vascular permeability reducing effect in a warm-blooded animal such as a human, may comprise administering to said animal an effective amount of AZD2171 or a pharmaceutically acceptable salt thereof, before, after or simultaneously with an effective amount of a taxane and before, after or simultaneously with an effective amount of ionising radiation.

A method for the production of an antiangiogenic and/or vascular permeability reducing effect in a warm-blooded animal such as a human, may comprise administering to said animal an effective amount of AZD2171, or a pharmaceutically acceptable salt thereof excluding an AZD2171 maleate salt, before, after or simultaneously with an effective amount of a taxane and before, after or simultaneously with an effective amount of ionising radiation.

A method for the treatment of a cancer in a warm-blooded animal such as a human, may comprise administering to said animal an effective amount of AZD2171 or a pharmaceutically acceptable salt thereof, before, after or simultaneously with an effective amount of a taxane and before, after or simultaneously with an effective amount of ionising radiation.

A method for the treatment of a cancer in a warm-blooded animal such as a human, may comprise administering to said animal an effective amount of AZD2171, or a pharmaceutically acceptable salt thereof excluding an AZD2171 maleate salt, before, after or simultaneously with an effective amount of a taxane and before, after or simultaneously with an effective amount of ionising radiation.

A method for the treatment of a cancer involving a solid tumour in a warm-blooded animal such as a human, may comprise administering to said animal an effective amount of AZD2171 or a pharmaceutically acceptable salt thereof, before, after or simultaneously with an effective amount of a taxane and before, after or simultaneously with an effective amount of ionising radiation.

A method for the treatment of a cancer involving a solid tumour in a warm-blooded animal such as a human, may comprise administering to said animal an effective amount of AZD2171, or a pharmaceutically acceptable salt thereof excluding an AZD2171 maleate salt, before, after or simultaneously with an effective amount of a taxane and before, after or simultaneously with an effective amount of ionising radiation.

A method for the production of an antiangiogenic and/or vascular permeability reducing effect in a warm-blooded animal such as a human, may comprise administering to said animal an effective amount of AZDZ171 or a pharmaceutically acceptable salt thereof, before, after or simultaneously with an effective amount of a taxane and before, after or simultaneously with an effective amount of ionising radiation, wherein AZD2171 and a taxane may each optionally be administered together with a pharmaceutically acceptable excipient or carrier.

A method for the production of an antiangiogenic and/or vascular permeability reducing effect in a warm-blooded animal such as a human, may comprise administering to said animal an effective amount of AZD2171, or a pharmaceutically acceptable salt thereof excluding an AZD2171 maleate salt, before, after or simultaneously with an effective amount of a taxane and before, after or simultaneously with an effective amount of ionising radiation, wherein AZD2171 and a taxane may each optionally be administered together with a pharmaceutically acceptable excipient or carrier.

A method for the treatment of a cancer in a warm-blooded animal such as a human, may comprise administering to said animal an effective amount of AZD2171 or a pharmaceutically acceptable salt thereof, before, after or simultaneously with an effective amount of a taxane and before, after or simultaneously with an effective amount of ionising radiation, wherein AZD2171 and a taxane may each optionally be administered together with a pharmaceutically acceptable excipient or carrier.

A method for the treatment of a cancer in a warm-blooded animal such as a human, may comprise administering to said animal an effective amount of AZD2171, or a pharmaceutically acceptable salt thereof excluding an AZD2171 maleate salt, before, after or simultaneously with an effective amount of a taxane and before, after or simultaneously with an effective amount of ionising radiation, wherein AZD2171 and a taxane may each optionally be administered together with a pharmaceutically acceptable excipient or carrier.

A method for the treatment of a cancer involving a solid tumour in a warm-blooded animal such as a human, may comprise administering to said animal an effective amount of A2D2171 or a pharmaceutically acceptable salt thereof, before, after or simultaneously with an effective amount of a taxane and before, after or simultaneously with an effective amount of ionising radiation, wherein AZD2171 and a taxane may each optionally be administered together with a pharmaceutically acceptable excipient or carrier.

A method for the treatment of a cancer involving a solid tumour in a warm-blooded animal such as a human, may comprise administering to said animal an effective amount of AZD2171, or a pharmaceutically acceptable salt thereof excluding an AZD2171 maleate salt, before, after or simultaneously with an effective amount of a taxane and before, after or simultaneously with an effective amount of ionising radiation, wherein AZD2171 and a taxane may each optionally be administered together with a pharmaceutically acceptable excipient or carrier.

According to a further aspect of the present invention there is provided the use of AZD2171 or a pharmaceutically acceptable salt thereof and a taxane in the manufacture of a medicament for use in the production of an antiangiogenic and/or vascular permeability reducing effect in a warm-blooded animal such as a human which is being treated with ionising radiation.

According to a further aspect of the present invention there is provided the use of AZD2171 or a pharmaceutically acceptable salt thereof excluding an AZD2171 maleate salt, and a taxane in the manufacture of a medicament for use in the production of an antiangiogenic and/or vascular permeability reducing effect in a warm-blooded animal such as a human which is being treated with ionising radiation.

According to a further aspect of the present invention there is provided the use of AZD2171 or a pharmaceutically acceptable salt thereof and a taxane in the manufacture of a medicament for use in the production of an anti-cancer effect in a warm-blooded animal such as a human which is being treated with ionising radiation.

According to a further aspect of the present invention there is provided the use of AZD2171 or a pharmaceutically acceptable salt thereof excluding an AZD2171 maleate salt, and a taxane in the manufacture of a medicament for use in the production of an anti-cancer effect in a warm-blooded animal such as a human which is being treated with ionising radiation.

According to a further aspect of the present invention there is provided the use of AZD2171 or a pharmaceutically acceptable salt thereof and a taxane in the manufacture of a medicament for use in the production of an anti-tumour effect in a warm-blooded animal such as a human which is being treated with ionising radiation.

According to a further aspect of the present invention there is provided the use of AZD2171 or a pharmaceutically acceptable salt thereof excluding an AZD2171 maleate salt, and a taxane in the manufacture of a medicament for use in the production of an anti-tumour effect in a warm-blooded animal such as a human which is being treated with ionising radiation.

According to a further aspect of the present invention there is provided a therapeutic combination treatment comprising the administration of an effective amount of AZD2171 or a pharmaceutically acceptable salt thereof, optionally together with a pharmaceutically acceptable excipient or carrier, and the administration of an effective amount of a taxane, optionally together with a pharmaceutically acceptable excipient or carrier and the administration of an effective amount of ionising radiation, to a warm-blooded animal such as a human in need of such therapeutic treatment wherein the AZD2171, taxane and ionising radiation may be administered simultaneously, sequentially or separately and in any order.

According to a further aspect of the present invention there is provided a therapeutic combination treatment comprising the administration of an effective amount of AZD2171 or a pharmaceutically acceptable salt thereof excluding an AZD2171 maleate salt, optionally together with a pharmaceutically acceptable excipient or carrier, and the administration of an effective amount of a taxane, optionally together with a pharmaceutically acceptable excipient or carrier and the administration of an effective amount of ionising radiation, to a warm-blooded animal such as a human in need of such therapeutic treatment wherein the AZD2171, taxane and ionising radiation may be administered simultaneously, sequentially or separately and in any order.

A warm-blooded animal such as a human which is being treated with ionising radiation means a warm-blooded animal such as a human which is treated with ionising radiation before, after or at the same time as the administration of a medicament or combination treatment comprising AZD2171 and a taxane. For example said ionising radiation may be given to said warm-blooded animal such as a human within the period of a week before to a week after the administration of a medicament or combination treatment comprising AZD2171 and a taxane. This means that AZD2171, a taxane and ionising radiation may be administered separately or sequentially in any order, or may be administered simultaneously. The warm-blooded animal may experience the effect of each of AZD2171, a taxane and radiation simultaneously.

According to one aspect of the present invention the ionising radiation is administered before one of AZD2171 and a taxane or after one of AZD2171 and a taxane.

According to one aspect of the present invention the ionising radiation is administered before both AZD2171 and a taxane or after both AZD2171 and a taxane.

According to one aspect of the present invention AZD2171 is administered to a warm-blooded animal after the animal has been treated with ionising radiation.

According to another aspect of the present invention the effect of a treatment of the present invention is expected to be at least equivalent to the addition of the effects of each of the components of said treatment used alone, that is, of each of AZD2171 and a taxane used alone or of each of AZD2171, a taxane and ionising radiation used alone.

According to another aspect of the present invention the effect of a treatment of the present invention is expected to be greater than the addition of the effects of each of the components of said treatment used alone, that is, of each of AZD2171 and a taxane used alone or of each of AZD2171, a taxane and ionising radiation used alone.

According to another aspect of the present invention the effect of a treatment of the present invention is expected to be a synergistic effect.

According to the present invention a combination treatment is defined as affording a synergistic effect if the effect is therapeutically superior, as measured by, for example, the extent of the response, the response rate, the time to disease progression or the survival period, to that achievable on dosing one or other of the components of the combination treatment at its conventional dose. For example, the effect of the combination treatment is synergistic if the effect is therapeutically superior to the effect achievable with AZD2171 or a taxane or ionising radiation alone. Further, the effect of the combination treatment is synergistic if a beneficial effect is obtained in a group of patients that does not respond (or responds poorly) to AZD2171 or a taxane or ionising radiation alone. In addition, the effect of the combination treatment is defined as affording a synergistic effect if one of the components is dosed at its conventional dose and the other component(s) is/are dosed at a reduced dose and the therapeutic effect, as measured by, for example, the extent of the response, the response rate, the time to disease progression or the survival period, is equivalent to that achievable on dosing conventional amounts of the components of the combination treatment. In particular, synergy is deemed to be present if the conventional dose of AZD2171 or a taxane or ionising radiation may be reduced without detriment to one or more of the extent of the response, the response rate, the time to disease progression and survival data, in particular without detriment to the duration of the response, but with fewer and/or less troublesome side-effects than those that occur when conventional doses of each component are used.

As stated above the combination treatments of the present invention as defined herein are of interest for their antiangiogenic and/or vascular permeability effects. Angiogenesis and/or an increase in vascular permeability is present in a wide range of disease states including cancer (including leukaemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, lymphoedema, endometriosis, dysfunctional uterine bleeding and ocular diseases with retinal vessel proliferation including age-related macular degeneration. Combination treatments of the present invention are expected to be particularly useful in the prophylaxis and treatment of diseases such as cancer and Kaposi's sarcoma.

Combination treatments of the present invention may be used to treat cancer, particularly a cancer involving a solid tumour. In particular such combination treatments of the invention are expected to slow advantageously the growth of primary and recurrent solid tumours of, for example, the colon, brain, thyroid, pancreas, bladder, breast, prostate, lungs and skin. More especially combination treatments of the present invention are expected to slow advantageously the growth of tumours in colorectal cancer and in lung cancer, for example mesothelioma and non-small cell lung cancer (NSCLC). More particularly such combination treatments of the invention are expected to inhibit any form of cancer associated with VEGF including leukaemia, multiple myeloma and lymphoma and also, for example, to inhibit the growth of those primary and recurrent solid tumours which are associated with VEGF, especially those tumours which are significantly dependent on VEGF for their growth and spread, including for example, certain tumours of the colon (including rectum), brain, thyroid, pancreas, bladder, breast, prostate, lung, vulva, skin and particularly NSCLC.

In another aspect of the present invention AZD2171 and a taxane, optionally with ionising radiation, are expected to inhibit the growth of those primary and recurrent solid tumours which are associated with VEGF especially those tumours which are significantly dependent on VEGF for their growth and spread.

The compositions described herein may be in a form suitable for oral administration, for example as a tablet or capsule, for nasal administration or administration by inhalation, for example as a powder or solution, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the AZD2171 of the combination treatment may be delivered endoscopically, intratracheally, intralesionally, percutaneously, intravenously, subcutaneously, intraperitoneally or intratumourally. Preferably AZD2171 is administered orally. In general the compositions described herein may be prepared in a conventional manner using conventional excipients. The compositions of the present invention are advantageously presented in unit dosage form.

AZD2171 will normally be administered to a warm-blooded animal at a unit dose within the range 1-50mg per square metre body area of the animal, for example approximately 0.03-1.5 mg/kg in a human. A unit dose in the range, for example, 0.01-1.5mg/kg, preferably 0.03-0.5mg/kg is envisaged and this is normally a therapeutically-effective dose. A unit dosage form such as a tablet or capsule will usually contain, for example 1-50mg of active ingredient. Preferably a daily dose in the range of 0.03-0.5mg/kg is employed.

Taxanes include paclitaxel and docetaxel. Paclitaxel and docetaxel are commerically available.

In one embodiment of the present invention a taxane is docetaxel.

In one embodiment of the present invention a taxane is paclitaxel.

A taxane may be dosed according to known routes of administration and dosages.

For example paclitaxel may be administered as an infusion over a period of about 24 hours at a dose of 135-200mg/m² every 3 weeks. Alternatively for example paclitaxel may be administered as an infusion over a period of about 3 hours at a dose of 135-225mg/m² every 3 weeks. Alternatively for example paclitaxel may be administered as an infusion over a period of about 1 hour at a dose of 80-100mg/m² every week for a number of weeks. Alternatively for example paclitaxel may be administered as an infusion over a period of about 1 hour at a dose of 200mg/m² every 3 weeks. Alternatively for example paclitaxel may be administered as an infusion over a period of about 96 hours at a dose of 120-140mg/m² every 3 weeks.

Docetaxel may be dosed in according with known routes of administration and dosages. For example docetaxel may be administered as an infusion over a period of 1 hour at a dose of 55-100mg/m² every 3 weeks.

Radiotherapy may be administered according to the known practices in clinical radiotherapy. The dosages of ionising radiation will be those known for use in clinical radiotherapy. The radiation therapy used will include for example the use of γ-rays, X-rays, and/or the directed delivery of radiation from radioisotopes. Other forms of DNA damaging factors are also included in the present invention such as microwaves and UV-irradiation. For example X-rays may be dosed in daily doses of 1.8-2.0Gy, 5 days a week for 5-6 weeks. Normally a total fractionated dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1Gy per hour over a number of days. Dosage ranges for radioisotopes vary widely, and depend on the half-life of the isotope, the strength and type of radiation emitted, and on the uptake by cells.

The size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatments in order to reduce toxicity.

The present invention relates to combinations of a taxane with AZD2171 or with a salt of AZD2171. A particular salt is an AZD2171 maleate salt.

In particular the present invention relates to combinations of a taxane with a form of the AZD2171 free base.

Salts of AZD2171 for use in pharmaceutical compositions will be pharmaceutically acceptable salts, but other salts may be useful in the production of AZD2171 and its pharmaceutically acceptable salts. Pharmaceutically acceptable salts may, for example, include acid addition salts. Such acid addition salts include for example salts with inorganic or organic acids affording pharmaceutically acceptable anions such as with hydrogen halides or with sulphuric or phosphoric acid, or with trifluoroacetic, citric or maleic acid. In addition pharmaceutically acceptable salts may be formed with an inorganic or organic base which affords a pharmaceutically acceptable cation. Such salts with inorganic or organic bases include for example an alkali metal salt, such as a sodium or potassium salt and an alkaline earth metal salt such as a calcium or magnesium salt.

AZD2171 may be synthesised according to the processes described in WO 00/47212, in particular those described in Example 240 of WO 00/47212.

AZD2171 maleate salt may be synthesised according to the processes described in International Patent Application No. PCT/GB2004/005359. For example, the following test may be used to demonstrate the activity of AZD2171 in combination with a taxane.

### MX-1 human breast tumour xenograft model

Tumour implantation procedures were performed on mice of at least 4 weeks of age. Human tumour xenografts were grown in female athymic (nu/nu genotype, Swiss) mice. MX-1 tumour fragments were implanted into athymic mice and allowed to grow to 0.7-1cm³ to provide donor tumour tissue. The donor tumours were surgically excised and smaller tumour fragments (20-30 mg) were implanted subcutaneously (s.c.) in the right flanks of the experimental athymic mice. Twenty days after tumour fragment implantation, when the mean tumour volume was 0.2cm³, randomisation was carried out (15 animals/group). Animals were treated with either docetaxel (10mg/kg, intravenously (i.v.) once weekly) or AZD2171 (1.5mg/kg or 3mg/kg, orally (p.o.) daily) or drug vehicle for the duration of the study. An additional group of animals (n=15) received a combination of docetaxel and AZD2171, using the same doses and schedules as used for single agent treatment. On days where animals received both AZD2171 and docetaxel the docetaxel was administered 2 hours after oral dosing with AZD2171.

Tumour volumes were assessed at least twice weekly by bilateral Vernier caliper measurement. Growth inhibition from the start of treatment was assessed by comparison of the differences in tumour volume between control and treated groups. The effects of combination treatment were assessed by comparing tumour growth in the group of animals receiving docetaxel plus AZD2171 with the tumour growth in the groups where animals received single agent therapy alone.

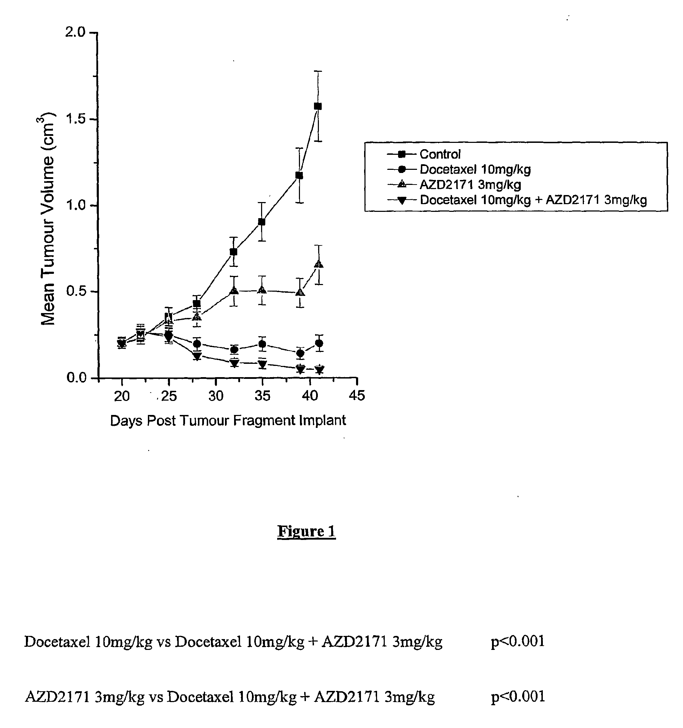

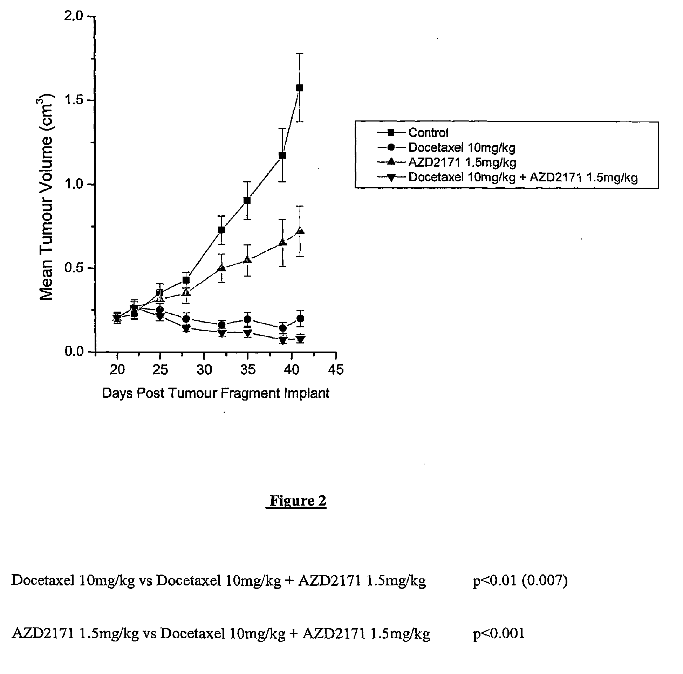

Statistical significance was evaluated using a one-tailed two-sample t-test. The data for combination studies wherein AZD2171 was dosed at 3 or 1.5mg/kg is shown in Figures 1 and 2. The growth inhibition of tumours was significantly greater with the combination of the two agents, AZD2171 and docetaxel, than with either agent alone.

An analogous experiment may be used to look at the combination of AZD2171, a taxane and ionising radiation.

## Claims

1. Use of AZD2171 or a pharmaceutically acceptable salt thereof excluding an AZD2171 maleate salt, and a taxane in the manufacture of a medicament for use in the production of an antiangiogenic and/or vascular permeability reducing effect in a warm-blooded animal such as a human.

2. Use of AZD2171 or a pharmaceutically acceptable salt thereof excluding an AZD2171 maleate salt, and a taxane in the manufacture of a medicament for use in the production of an anti-cancer effect in a warm-blooded animal such as a human.

3. Use of AZD2171 or a pharmaceutically acceptable salt thereof excluding an AZD2171 maleate salt, and a taxane in the manufacture of a medicament for use in the production of an anti-tumour effect in a warm-blooded animal such as a human.

4. Use of AZD2171 or a pharmaceutically acceptable salt thereof excluding an AZD2171 maleate salt, and a taxane in the manufacture of a medicament for use in the production of an antiangiogenic and/or vascular permeability reducing effect in a warm-blooded animal such as a human which is being treated with ionising radiation.

5. Use of AZD2171 or a pharmaceutically acceptable salt thereof excluding an AZD2171 maleate salt, and a taxane in the manufacture of a medicament for use in the production of an anti-cancer effect in a warm-blooded animal such as a human which is being treated with ionising radiation.

6. Use of AZD2171 or a pharmaceutically acceptable salt thereof excluding an AZD2171 maleate salt, and a taxane in the manufacture of a medicament for use in the production of an anti-tumour effect in a warm-blooded animal such as a human which is being treated with ionising radiation.

7. Use according to any one of claims 1-6 wherein AZD2171 is in a form of the free base.

8. Use according to any one of claims 1-7 wherein the taxane is paclitaxel.

9. Use according to any one of claims 1-7 wherein the taxane is docetaxel.

10. A pharmaceutical composition which comprises AZD2171 or a pharmaceutically acceptable salt thereof excluding an AZD2171 maleate salt, and a taxane in association with a pharmaceutically acceptable excipient or carrier.

11. A kit comprising AZD2171 or a pharmaceutically acceptable salt thereof and a taxane.

## Patentansprüche

1. Verwendung von AZD2171 oder einem pharmazeutisch annehmbaren Salz davon unter Ausschluß eines AZD2171-Maleatsalzes und einem Taxan bei der Herstellung eines Arzneimittels zur Erzielung einer antiangiogenen und/oder gefäßpermeabilitätssenkenden Wirkung bei einem Warmblüter wie einem Menschen.

2. Verwendung von AZD2171 oder einem pharmazeutisch annehmbaren Salz davon unter Ausschluß eines AZD2171-Maleatsalzes und einem Taxan bei der Herstellung eines Arzneimittels zur Erzielung einer Antikrebswirkung bei einem Warmblüter wie einem Menschen.

3. Verwendung von AZD2171 oder einem pharmazeutisch annehmbaren Salz davon unter Ausschluß eines AZD2171-Maleatsalzes und einem Taxan bei der Herstellung eines Arzneimittels zur Erzielung einer Antitumorwirkung bei einem Warmblüter wie einem Menschen.

4. Verwendung von AZD2171 oder einem pharmazeutisch annehmbaren Salz davon unter Ausschluß eines AZD2171-Maleatsalzes und einem Taxan bei der Herstellung eines Arzneimittels zur Erzielung einer antiangiogenen und/oder gefäßpermeabilitätssenkenden Wirkung bei einem Warmblüter wie einem Menschen, der mit ionisierender Strahlung behandelt wird.

5. Verwendung von AZD2171 oder einem pharmazeutisch annehmbaren Salz davon unter Ausschluß eines AZD2171-Maleatsalzes und einem Taxan bei der Herstellung eines Arzneimittels zur Erzielung einer Antikrebswirkung bei einem Warmblüter wie einem Menschen, der mit ionisierender Strahlung behandelt wird.

6. Verwendung von AZD2171 oder einem pharmazeutisch annehmbaren Salz davon unter Ausschluß eines AZD2171-Maleatsalzes und einem Taxan bei der Herstellung eines Arzneimittels zur Erzielung einer Antitumorwirkung bei einem Warmblüter wie einem Menschen, der mit ionisierender Strahlung behandelt wird.

7. Verwendung nach einem der Ansprüche 1-6, bei der AZD2171 in Form der freien Base vorliegt.

8. Verwendung nach einem der Ansprüche 1-7, bei der es sich bei dem Taxan um Paclitaxel handelt.

9. Verwendung nach einem der Ansprüche 1-7, bei der es sich bei dem Taxan um Docetaxel handelt.

10. Pharmazeutische Zusammensetzung, enthaltend AZD2171 oder ein pharmazeutisch annehmbares Salz davon unter Ausschluß eines AZD2171-Maleatsalzes und ein Taxan zusammen mit einem pharmazeutisch annehmbaren Hilfsstoff oder Träger.

11. Kit, enthaltend AZD2171 oder ein pharmazeutisch annehmbares Salz davon und ein Taxan.

## Revendications

1. Utilisation d'AZD2171 ou d'un sel pharmaceutiquement acceptable de celui-ci à l'exclusion d'un sel maléate d'AZD2171, et d'un taxane dans la fabrication d'un médicament destiné à être utilisé dans la production d'un effet anti-angiogénique et/ou réducteur de la perméabilité vasculaire chez un animal à sang chaud tel que l'homme.

2. Utilisation d'AZD2171 ou d'un sel pharmaceutiquement acceptable de celui-ci à l'exclusion d'un sel maléate d'AZD2171, et d'un taxane dans la fabrication d'un médicament destiné à être utilisé dans la production d'un effet anticancéreux chez un animal à sang chaud tel que l'homme.

3. Utilisation d'AZD2171 ou d'un sel pharmaceutiquement acceptable de celui-ci à l'exclusion d'un sel maléate d'AZD2171, et d'un taxane dans la fabrication d'un médicament destiné à être utilisé dans la production d'un effet antitumoral chez un animal à sang chaud tel que l'homme.

4. Utilisation d'AZD2171 ou d'un sel pharmaceutiquement acceptable de celui-ci à l'exclusion d'un sel maléate d'AZD2171, et d'un taxane dans la fabrication d'un médicament destiné à être utilisé dans la production d'un effet anti-angiogénique et/ou réducteur de la perméabilité vasculaire chez un animal à sang chaud tel que l'homme traité par des radiations ionisantes.

5. Utilisation d'AZD2171 ou d'un sel pharmaceutiquement acceptable de celui-ci à l'exclusion d'un sel maléate d'AZD2171, et d'un taxane dans la fabrication d'un médicament destiné à être utilisé dans la production d'un effet anticancéreux chez un animal à sang chaud tel que l'homme traité par des radiations ionisantes.

6. Utilisation d'AZD2171 ou d'un sel pharmaceutiquement acceptable de celui-ci à l'exclusion d'un sel maléate d'AZD2171, et d'un taxane dans la fabrication d'un médicament destiné à être utilisé dans la production d'un effet antitumoral chez un animal à sang chaud tel que l'homme traité par des radiations ionisantes.

7. Utilisation selon l'une quelconque des revendications 1 à 6, **caractérisée en ce qu'**AZD2171 est sous forme de base libre.

8. Utilisation selon l'une quelconque des revendications 1 à 7, **caractérisée en ce que** le taxane est le paclitaxel.

9. Utilisation selon l'une quelconque des revendications 1 à 7, **caractérisée en ce que** le taxane est le docétaxel.

10. Composition pharmaceutique qui comprend l'AZD2171 ou un sel pharmaceutiquement acceptable de celui-ci à l'exclusion d'un sel maléate d'AZD2171, et un taxane en association avec un excipient ou un support pharmaceutiquement acceptable.

11. Kit comprenant l'AZD2171 ou un sel pharmaceutiquement acceptable de celui-ci et un taxane.
